# EUROPÄISCHE PATENTSCHRIFT

(11) **EP 4 454 617 B1**
(45) Veröffentlichungstag und Bekanntmachung des Hinweises auf die Patenterteilung: **08.07.2026**
(21) Anmeldenummer: 24171006.0
(22) Anmeldetag: 18.04.2024
(51) Int. Cl.: A61F 2/46, A61F 2/30, A61F 2/38, B33Y 80/00

(54) **TIBIALES PROBEIMPLANTAT**
TIBIAL TRIAL IMPLANT
IMPLANT TIBIAL POUR SONDE

(30) Priorität: 26.04.2023 DE 102023110667
(43) Veröffentlichungstag der Anmeldung: 30.10.2024
(73) Patentinhaber: Aesculap AG, 78532 Tuttlingen (DE)
(72) Erfinder: Hettich, Georg, 79117 Freiburg (DE); Mayrhofer, Lucy, 72144 Dusslingen (DE)
(74) Vertreter: Patentanwälte Ruff, Wilhelm, Beier, Dauster & Partner mbB

(56) Entgegenhaltungen:
- WO-A1-2012/004580
- US-A- 5 865 848
- US-A1- 2020 289 291
- US-B2- 10 188 530

## Beschreibung

Die Erfindung betrifft ein tibiales Probeimplantat zur Verwendung bei einer Kniegelenkersatzoperation.

Bei einer Kniegelenkersatzoperation (englisch: total knee athroplasty (TKA)) werden Gelenkflächen des Femurs und der Tibia durch künstliche Gelenkflächen einer Kniegelenkprothese ersetzt. Kniegelenkprothesen umfassen üblicherweise ein Femurimplantat, ein Tibiaimplantat und eine Meniskuskomponente, die oftmals auch als Gleitfläche bezeichnet wird. Das Femurimplantat wird am distalen Ende des Femurs implantiert. Das Tibiaimplantat wird am proximalen Ende der Tibia implantiert. Die Meniskuskomponente ist proximodistal zwischen Tibia- und Femurimplantat angeordnet. Um eine einwandfreie Funktion des künstlichen Gelenkersatzes zu gewährleisten, müssen die besagten Implantate hinsichtlich ihrer Lage und Orientierung in Bezug auf die Körperachsen des Patienten in definierter Weise präzise positioniert werden. Zusätzlich zu der präzisen Positionierung ist es wichtig, dass die Implantate jeweils mit für die Anatomie des Patienten passenden Abmessungen ausgewählt und implantiert werden. Hinsichtlich der Meniskuskomponente ist insbesondere deren proximodistale Höhe ein wesentliches Kriterium.

Das tibiale Probeimplantat dient einer intraoperativen Ermittlung der erforderlichen proximodistalen Höhe der zu implantierenden Meniskuskomponente. Im Speziellen wird das tibiale Probeimplantat verwendet, um bei bereits implantiertem Femurimplantat eine optimale Bandspannung und Gelenklinie zu testen. Abhängig vom Ergebnis dieser Testung wird die Meniskuskomponente ausgewählt und implantiert. Die besagte Testung kann beispielsweise anhand unterschiedlicher Meniskusprobekomponenten mit unterschiedlichen definierten Höhen erfolgen.

Aus der EP 4 011 334 A1 ist ein tibiales Probeimplantat mit einer oberen Platte und einer unteren Platte bekannt. Die obere Platte weist eine proximale Artikulationsfläche zur Artikulation mit einer femoralen Komponente auf. Die untere Platte weist eine distale Lagerfläche zur Lagerung auf einem Tibiaplateau auf. Zwischen der oberen Platte und der unteren Platte ist ein Höheneinstellmechanismus angeordnet. Der Höheneinstellmechanismus dient einer Einstellung eines proximodistalen Abstands zwischen der Artikulationsfläche der oberen Platte und der Lagerfläche der unteren Platte und damit der proximodistalen Höhe des Probeimplantats. Der Höheneinstellmechanismus weist zwei miteinander gekoppelte Scherengelenkmechanismen auf.

Aus der DE 10 2020 208 501 A1 ist ein weiteres tibiales Probeimplantat bekannt. Das bekannte tibiale Probeimplantat weist ein Oberteil mit einer proximal angeordneten Gleitfläche zum gleitenden Zusammenwirken mit einer femoralen Komponente auf. Das Unterteil ist zur tibialen Fixierung vorgesehen. Zwischen dem Oberteil und dem Unterteil ist ein Höheneinstellmechanismus angeordnet, mittels dessen das Oberteil relativ zu dem Unterteil in Hochrichtung geführt verlagerbar ist. Die Verlagerung des Oberteils dient wiederum der Einstellung des besagten proximodistalen Abstands und damit der Höhe des Probeimplantats. Der Höheneinstellmechanismus weist ein Kurvengetriebe mit einem rotierenden Antriebsrad auf.

Aus der EP 4 082 485 A1 ist ein weiteres tibiales Probeimplantat bekannt. Dieses Probeimplantat weist eine Lagerkomponente mit einer proximalen Artikulationsfläche und einer distalen Fläche auf. Zudem ist eine Plattenkomponente mit einer proximalen Oberfläche und einer distalen Befestigungsoberfläche vorhanden. Zwischen der Lagerkomponente und der Plattenkomponente ist eine Abstandseinstellanordnung angeordnet, die einerseits lösbar mit der Lagerkomponente und andererseits lösbar mit der Plattenkomponente in Eingriff kommt und die beiden Komponenten in proximodistaler Richtung begrenzt beweglich miteinander koppelt. Weiter weist das bekannte tibiale Probeimplantat mehrere Abstandsstücke (englisch: shims) auf, die zur Einstellung der proximodistalen Höhe zwischen die Lager- und die Plattenkomponente in die Abstandseinstellanordnung einschiebbar sind. Unterschiedliche Abstandsstücke weisen unterschiedliche proximodistale Dicken auf. Je nach Auswahl des Abstandsstücks kann eine unterschiedliche proximodistale Höhe des Probeimplantats eingestellt werden.

Aus der WO 2012/004580 A1 ist ein chirurgisches Instrument bekannt, das zwei Spacer-Block-Teile aufweist, die jeweils eine erste und eine geneigte zweite Fläche aufweisen. Diese Teile sind so miteinander verbunden, dass die zweiten Flächen in Kontakt stehen und parallel zueinander gleiten können, um die Dicke des Instruments anzupassen. Die zweiten Flächen enthalten Strukturen, die beim Zusammenwirken eine ungewollte Gleitbewegung zwischen den Block-Teilen verhindern.

Aus der US 10 188 530 B2 ist ein tibiales Probeprothesensystem bekannt, das eine Lagereinheit und eine Lagersupporteinheit umfasst, deren Abstand verstellbar ist, um verschiedene Größen finaler Prothesen darzustellen. Nur eine Lagereinheit pro Einschränkungsgrad ist erforderlich, während Unterlegscheiben den Abstand anpassen. Diese Unterlegscheiben werden in einer anteroposterioren Richtung zwischen der Lagereinheit und der Lagersupporteinheit eingeführt.

Aus der US 2020/0289291 A1 ist eine Endoprothese bekannt, die ein erstes Element, das mit dem Knochen des Patienten verbunden ist, und ein zweites Element aufweist, das verstellbar gekoppelt durch eine Kupplungseinrichtung zu dem ersten Element relativbeweglich ist. Außerdem wird ein künstliches Gelenk mit einer solchen Endoprothese beschrieben, ebenso wie ein Platzierungssystem, das eine Projektionsvorrichtung umfasst.

Aufgabe der Erfindung ist es, ein tibiales Probeimplantat bereitzustellen, das einen gegenüber dem Stand der Technik vereinfachten Aufbau aufweist und eine einfache Anwendung erlaubt.

Diese Aufgabe wird durch das Bereitstellen eines tibialen Probeimplantats mit den Merkmalen des Anspruchs 1 gelöst.

Das erfindungsgemäße tibiale Probeimplantat weist eine Artikulationskomponente und wenigstens eine Stützkomponente auf. Die Artikulationskomponente weist eine proximale Artikulationsfläche und eine distale Unterseite auf. Die proximale Artikulationsfläche ist zur Artikulation mit einer femoralen Komponente eingerichtet. Die wenigstens eine Stützkomponente weist eine distale Lagerfläche, eine proximale Oberseite und eine Führungseinrichtung auf. Die distale Lagerfläche ist zur Lagerung auf einem Tibiaplateau eingerichtet. Die proximale Oberseite bildet eine relativ zu der distalen Lagerfläche geneigte Rampe. Die Führungseinrichtung weist eine parallel zu der Rampe längserstreckte Führungsbahn auf. Die Unterseite der Artikulationskomponente weist eine parallel zu der Rampe geneigte Stützfläche auf. Die Artikulationskomponente ist an der Führungseinrichtung gehalten und entlang der Führungsbahn relativ zu der Stützkomponente linearbeweglich mit ihrer Stützfläche auf der Rampe abgestützt. Eine Relativbewegung zwischen der Artikulationskomponente und der Stützkomponente bewirkt folglich eine Veränderung eines proximodistalen Abstands zwischen der Artikulationsfläche der Artikulationskomponente und der Lagerfläche der Stützkomponente. Durch die erfindungsgemäße Lösung weist das tibiale Probeimplantat einen besonders einfachen Aufbau auf. Insbesondere kann auf komplex aufgebaute Höheneinstellmechanismen und auf die Verwendung unterschiedlicher Meniskusprobekomponenten mit unterschiedlichen Höhen verzichtet werden. Stattdessen ist es vorgesehen, dass die wenigstens eine Stützkomponente nach Art eines Keils zwischen das Tibiaplateau und die Artikulationskomponente geschoben werden kann. Zu diesem Zweck ist die Oberseite der Stützkomponente relativ zu der distalen Lagerfläche geneigt und bildet die besagte Rampe. Die distale Unterseite der Artikulationskomponente ist zu der Rampe parallel längserstreckt und folglich dementsprechend geneigt. Um undefinierte Relativbewegungen zwischen der Artikulationskomponente und der Stützkomponente zu unterbinden, ist die Führungseinrichtung vorgesehen. Die Führungseinrichtung legt die Artikulationskomponente und die Stützkomponente entlang der parallel zu der Rampe - und damit auch der Stützfläche - erstreckten Führungsbahn linearbeweglich aneinander fest. Eine Relativbewegung zwischen der Artikulationskomponente und der Stützkomponente entlang der Führungsbahn bewirkt folglich eine Veränderung des proximodistalen Abstands. Je größer die Relativbewegung, desto größer die Veränderung des Abstands. Ausgehend von einer (intraoperativen) Situation, in welcher die Artikulationskomponente örtlich festgelegt ist, kann die Stützkomponente mehr oder weniger weit zwischen die Artikulationskomponente und das Tibiaplateau eingeschoben werden. In Abhängigkeit des Vorschubs der Stützkomponente wird die Artikulationskomponente mehr oder weniger weit proximal in Richtung der femoralen Komponente verlagert und damit der proximodistale Abstand zwischen der Lagerfläche der Stützkomponente - und damit dem Tibiaplateau - und der Artikulationsfläche verändert. Die femorale Komponente und das Tibiaplateau sind nicht Bestandteil des tibialen Probeimplantats. Bei der femoralen Komponente kann es sich um einen natürlichen distalen Femur oder eine femorale Implantatskomponente handeln. Das Tibiaplateau kann durch eine Resektionsfläche an der proximalen Tibia oder eine künstliche Plateaufläche, die an der Resektionsfläche befestigt ist, gebildet sein. Die zur Lagerung auf dem Tibiaplateau eingerichtete Lagerfläche ist vorzugsweise plan und parallel zu einer anteroposterior und mediolateral erstreckten Transversalebene. Die durch die Oberseite der Stützkomponente gebildete Rampe ist gegenüber der Transversalebene geneigt, so dass sich, insbesondere bei einer mediolateralen oder anteroposterioren Blickrichtung, eine keilförmige Gestalt der Stützkomponente ergibt. Bei einer Relativbewegung zwischen der Tibiaund der Stützkomponente gleiten die Stützfläche der Artikulationskomponente und die Rampe aneinander ab. Gleichzeitig erfolgt eine Führung der beiden Komponenten mittels der Führungseinrichtung. Die Führungseinrichtung ist wenigstens abschnittsweise an der Stützkomponente angeordnet und/oder ausgebildet und wirkt zwecks Führung mit einem hierfür eingerichteten Abschnitt der Artikulationskomponente zusammen. Bei einer Ausgestaltung weist das tibiale Probeimplantat exakt eine Stützkomponente auf, so dass ein insgesamt zweiteiliger Aufbau vorgesehen ist. Bei einer weiteren Ausgestaltung weist das tibiale Probeimplantat exakt zwei Stützkomponenten auf, so dass ein insgesamt dreiteiliger Aufbau des Probeimplantats vorgesehen ist.

Die in dieser Beschreibung verwendeten Lage- und Richtungsbezeichnungen beziehen sich auf den Körper eines Patienten, insbesondere dessen Tibia, und sind gemäß ihrer üblichen anatomischen Bedeutung zu verstehen. Folglich bedeutet "anterior" vordere/r oder vorne liegend, "posterior" hintere/r oder hinten liegend, "medial" innere/r oder innen liegend, "lateral" äußere/r oder außen liegend, "proximal" zum Körperzentrum hin oder oben liegend, "distal" vom Körperzentrum entfernt oder unten liegend. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander ausgerichtet und können selbstverständlich in Bezug zu nicht mit der Anatomie des Patienten in Verbindung stehenden X-, Y- und Z-Achsen gesetzt werden. Beispielsweise kann die proximodistale Achse alternativ als Z-Achse bezeichnet werden. Die medio-laterale Achse kann als Y-Achse bezeichnet werden. Die anterior-posteriore Achse kann als X-Achse bezeichnet werden. Zur besseren Veranschaulichung und der Einfachheit der Bezeichnungen wegen werden nachfolgend in erster Linie die besagten anatomischen Lage- und Richtungsbezeichnungen verwendet. Weiter werden Bezeichnungen wie "Oberseite" in Bezug auf eine distal gerichtete Blickrichtung verwendet. Dementgegen werden Bezeichnungen wie "Unterseite" in Bezug auf eine proximal gerichtete Blickrichtung verwendet.

In Ausgestaltung der Erfindung ist die Rampe und/oder die Stützfläche plan und weist eine Oberflächenprofilierung auf, die dazu eingerichtet ist, einem Abrutschen der Artikulationskomponente von der Rampe entgegenzuwirken. Hierdurch wird einer ungewollten Veränderung eines einmal eingestellten proximodistalen Abstands entgegengewirkt. Die Oberflächenprofilierung bewirkt eine erhöhte Reibung zwischen der Rampe und der Stützfläche. Die Oberflächenprofilierung weist vorzugsweise in Normalenrichtung der Rampe und/oder Stützfläche aufragende Erhebungen und/oder eingebrachte Vertiefungen auf. Durch die plane Gestaltung der Rampe und/oder der Stützfläche ist vorzugsweise eine stufenlose Veränderung des proximodistalen Abstands möglich.

In weiterer Ausgestaltung der Erfindung sind die Rampe und die Stützfläche jeweils mit einer Abfolge von Stufen versehen, wodurch die Relativbewegung zwischen der Artikulationskomponente und der Stützkomponente eine stufenweise Veränderung des proximodistalen Abstands bewirkt. Die Stufen weisen jeweils eine in proximodistaler Richtung erstreckte Höhe auf. Die Stufen steigen ausgehend von einem (niedrigen) Ende der Rampe in Richtung eines gegenüberliegenden (hohen) Endes an. Entsprechendes gilt, mutatis mutandis, für die Stufen der Stützfläche. Vorzugsweise sind die Stufen jeweils angeschrägt, so dass die Stützfläche und die Rampe - trotz vorhandener Stufen - entlang der Führungsbahn aneinander abgleiten können.

In weiterer Ausgestaltung der Erfindung weisen die Stufen jeweils eine proximodistale Höhe zwischen 1 mm und 3 mm, bevorzugt 2 mm, auf, wobei jeweils wenigstens vier Stufen vorhanden sind. Hierdurch ergibt sich ein wenigstens 4-stufiger Einstellbereich für den proximodistalen Abstand. Dabei hat sich eine Höhe von 2 mm für die Stufen als besonders vorteilhaft erwiesen. Die genannte Höhe ist einerseits ausreichend klein, um auch geringe Veränderungen des Abstands einstellen zu können. Andererseits erlaubt eine Höhe von 2 mm pro Stufe bei wenigstens vier Stufen einen insgesamt ausreichend großen Einstellbereich. Die Höhe der einzelnen Stufen und die realisierbare Gesamthöhe des tibiales Probeimplantats orientiert sich vorzugsweise an den verfügbaren, unterschiedlich hohen Meniskuskomponenten, die in der Regel in den Höhen 10 mm, 12 mm, 14 mm, 16 mm, 18 mm, 20 mm, 22 mm und 24 mm verfügbar sind.

In weiterer Ausgestaltung der Erfindung weisen die Stufen der Rampe jeweils eine konvexe Auswölbung auf und die Stufen der Stützfläche weisen jeweils eine komplementäre konkave Einwölbung auf, wobei die Auswölbungen und die Einwölbungen lösbar ineinandergreifen, wodurch einer ungewollten Relativbewegung zwischen der Artikulationskomponente und der Stützkomponente entgegengewirkt ist. Durch das Ineinandergreifen der Aus- und Einwölbungen wird ungewollten Relativbewegungen zwischen den komplementären Stufen und damit zwischen der Artikulationskomponente und der Stützkomponente entgegengewirkt. Hierdurch werden ungewollte Höhenveränderungen des tibialen Probeimplantats vermieden. Zur gewollten Relativbewegung kann der Eingriff zwischen den Einwölbungen und den Auswölbungen manuell überwunden werden, beispielweise indem die Artikulationskomponente geringfügig von der Stützkomponente angehoben wird. Es versteht sich, dass auch eine umgekehrte Anordnung von Auswölbungen und Einwölbungen vorgesehen sein kann. Folglich sind bei einer Ausgestaltung die konkaven Einwölbungen an den Stufen der Rampe und die konvexen Auswölbungen an den Stufen der Stützfläche vorhanden.

In weiterer Ausgestaltung der Erfindung weist die Rampe und/oder die Stützfläche eine Längsneigung von 10° bis 45°, bevorzugt von 15° bis 30°, besonders bevorzugt von 20°, auf. Die Längsneigung bemisst sich relativ zu der Lagerfläche und damit auch dem Tibiaplateau. Alternativ oder zusätzlich bemisst die Längsneigung sich relativ zu einer gedachten Transversalebene. Je steiler die Längsneigung ist, desto größer ist ein Übersetzungsverhältnis zwischen der Relativbewegung der Tibia- und der Stützkomponente und der Veränderung des proximodistalen Abstands. Eine kleine Relativbewegung bewirkt folglich eine in Relation große Veränderung des proximodistalen Abstands. Gleichzeitig ist hierbei ein relativ großer Kraftaufwand zum Vorschub der Stützkomponente erforderlich. Umgekehrtes gilt, mutatis mutandis, für flache Längsneigungen. Vor diesem Hintergrund hat sich eine Längsneigung in dem Bereich von 15 bis 30° als vorteilhaft erwiesen, wobei 20° als Optimum angesehen werden kann.

In weiterer Ausgestaltung der Erfindung sind die Rampe und die Stützfläche anteroposterior längsgeneigt. Mit anderen Worten erhebt die Rampe sich entlang einer anteroposterioren Achse, wobei Entsprechendes sinngemäß für die Stützfläche gilt. Da die Führungsbahn parallel zu der Rampe und damit auch der Stützfläche längserstreckt ist, gilt das Gesagte auch für deren Längsneigung. Bei dieser Ausgestaltung wird die wenigstens eine Stützkomponente zur Veränderung des proximodistalen Abstands relativ zu der Artikulationskomponente anteroposterior verlagert. Vorzugsweise bewirkt ein von anterior nach posterior gerichteter relativer Vorschub der Stützkomponente eine Vergrößerung des proximodistalen Abstands. Eine Verringerung erfolgt in kinematisch umgekehrter Weise.

In weiterer Ausgestaltung der Erfindung sind die Rampe und die Stützfläche mediolateral längsgeneigt. Die Rampe steigt folglich entlang einer mediolateralen Achse an. Dies gilt, mutatis mutandis, auch für die Stützfläche. Bei dieser Ausgestaltung kann die wenigstens eine Stützkomponente zur intraoperativen Veränderung des proximodistalen Abstands relativ zu der Artikulationskomponente in mediolateraler Richtung verlagert werden. Mit anderen Worten ist ein "seitlicher" relativer Vorschub der Stützkomponente vorgesehen. Sofern das tibiale Probeimplantat exakt eine Stützkomponente aufweist, kann diese bei dieser Ausgestaltung der Erfindung lateral oder medial einer sagittalen Mittellängsebene der Artikulationskomponente angeordnet sein. Ist die Stützkomponente lateral angeordnet, bewirkt ein medialer Vorschub vorzugsweise eine Vergrößerung des proximodistalen Abstands. Ist die Stützkomponente stattdessen medial angeordnet, bewirkt vorzugsweise ein lateraler Vorschub eine Vergrößerung des proximodistalen Abstands. Für eine Verringerung des proximodistalen Abstands wird die Stützkomponente kinematisch entgegengesetzt verlagert.

In weiterer Ausgestaltung der Erfindung sind zwei Stützkomponenten vorhanden und beidseits einer sagittalen Mittellängsebene der Artikulationskomponente angeordnet, wobei die beiden Stützkomponenten zur Veränderung des proximodistalen Abstands mediolateral entgegengesetzt relativ zu der Artikulationskomponente beweglich sind. Bei dieser Ausgestaltung kann auch von einer ersten Stützkomponente und einer zweiten Stützkomponente gesprochen werden. Die erste Stützkomponente ist vorzugsweise auf einer lateralen Seite der sagittalen Mittellängsebene angeordnet. Die zweite Stützkomponente ist vorzugsweise auf einer medialen Seite der sagittalen Mittellängsebene angeordnet. Zur Veränderung des proximodistalen Abstands werden die beiden Stützkomponenten zueinander entgegengesetzt relativ zu der Artikulationskomponente bewegt. Diese Ausgestaltung erlaubt insbesondere eine medial und lateral quantitativ unterschiedliche Veränderung des proximodistalen Abstands. Beispielsweise kann die Artikulationsfläche auf einer medialen Seite weiter proximal angehoben werden als auf einer lateralen Seite oder umgekehrt. Hierdurch kann der Anatomie des Patienten in verbesserter Weise Rechnung getragen werden.

In weiterer Ausgestaltung der Erfindung weist die Führungseinrichtung wenigstens eine Führungskulisse auf, in welche wenigstens ein Führungsstift der Artikulationskomponente entlang der Führungsbahn gleitbeweglich eingreift. Die Führungskulisse ist in die Stützkomponente eingebracht und definiert die Führungsbahn. Sofern die Rampe und die Stützfläche jeweils mit einer Abfolge von Stufen versehen sind, gilt dies vorzugsweise auch für die Führungskulisse. In diesem Fall sind die Stufen der Führungskulisse parallel versetzt zu den Stufen der Rampe. Der wenigstens eine Führungsstift ist an der Artikulationskomponente angeordnet und/oder ausgebildet. Der Führungsstift greift entlang der Führungsbahn beweglich in die Führungskulisse ein. Bei einer Ausgestaltung ist die Führungskulisse einends offen und weist an ihrem offenen Ende eine Einführöffnung für die Führungsstift auf. Zwecks Montage des tibialen Probeimplantats kann der Führungsstift durch die Einführöffnung in die Führungskulisse eingeführt werden. Bei einer weiteren Ausgestaltung ist die Führungskulisse an ihren gegenüberliegenden Enden geschlossen. Zwecks Montage kann der Führungsstift bei dieser Ausgestaltung relativ zu der Artikulationskomponente verlagerbar sein. Alternativ kann eine additive Fertigung gemäß einer nachfolgend noch näher beschriebenen Ausgestaltung vorgesehen sein. Bei einer Ausgestaltung sind zwei zueinander parallel erstreckte Führungskulissen und dementsprechend zwei Führungsstifte vorgesehen.

In weiterer Ausgestaltung der Erfindung weist die Führungskulisse einends der Führungsbahn eine Einführöffnung aufweist, die ausgehend von der Lagerfläche im Wesentlichen senkrecht zu der Führungsbahn in die Führungskulisse mündet und durch welche der wenigstens eine Führungsstift in die Führungskulisse einführbar ist. Zum Einführen des Führungsstifts kann die Artikulationskomponente mit einer gegeneinander vertauschten Ausrichtung ihrer Artikulationsfläche und ihrer Unterseite, d.h. mit etwa proximal ausgerichteter Unterseite, an der Stützkomponente angesetzt werden. Nach Einführen des wenigstens einen Führungsstifts in die Führungskulisse kann die Artikulationskomponente um 180° gedreht werden, so dass die Artikulationsfläche und die Unterseite ihre vorgesehene Ausrichtung wieder einnehmen. Durch diese spezielle Montage oder auch "Einführbewegung" ist die Beweglichkeit zwischen der Artikulationskomponente und der Stützkomponente nur entlang der Führungsbahn möglich. Hierdurch wird ein ungewolltes Trennen der Komponenten, insbesondere in anteroposteriorer und/oder in proximodistaler Richtung, verhindert.

In weiterer Ausgestaltung der Erfindung ist die Stützfläche proximal in die Unterseite der Artikulationskomponente eingesenkt und durch gegenüberliegende Wandabschnitte begrenzt, die jeweils senkrecht zu der Führungsbahn und senkrecht zu einer proximodistalen Achse formschlüssig an der Stützkomponente anliegen. Durch den besagten Formschluss wird eine nochmals verbesserte Führung der Relativbewegung zwischen der Tibia- und der Stützkomponente erreicht. Die Wandabschnitte liegen jeweils seitlich an der Stützkomponente, vorzugsweise der Rampe, an.

In weiterer Ausgestaltung der Erfindung weist die Artikulationskomponente eine proximodistal von der Artikulationsfläche bis zu der Stützfläche erstreckte Aussparung auf, welche die Artikulationsfläche und die Stützfläche in jeweils einen medialen Flächenabschnitt und einen lateralen Flächenabschnitt teilt. Bei dieser Ausgestaltung weist die Artikulationsfläche folglich einen medialen Artikulationsflächenabschnitt und einen lateralen Artikulationsflächenabschnitt auf. Diese beiden Abschnitte sind beidseits der Aussparung einander gegenüberliegend angeordnet und um eine mediolaterale Abmessung der Aussparung voneinander beabstandet. Entsprechendes gilt, mutatis mutandis, für die Stützfläche, so dass ein medialer Stützflächenabschnitt und ein lateraler Stützflächenabschnitt vorhanden sind. Die Aussparung erlaubt insbesondere eine verbesserte Einsehbarkeit der distal/unterhalb der Artikulationskomponente angeordneten Stützkomponente. Sofern die Stützkomponente ebenfalls mit einer Aussparung versehen ist, kann das Tibiaplateau eingesehen werden. Dies erlaubt dem ausführenden Operateur eine verbesserte intraoperative Kontrolle.

In weiterer Ausgestaltung der Erfindung weist die wenigstens eine Stützkomponente eine proximodistal durch die Rampe erstreckte Aussparung auf, welche die Rampe in zwei Rampenabschnitte teilt, die seitlich, insbesondere mediolateral oder anteroposterior, voneinander beabstandet sind. Die durch die Aussparung erstreckte Rampe ermöglicht zum einen eine Einsparung von Material und damit einhergehend von Fertigungsaufwand und Gewicht. Sofern die Artikulationskomponente gemäß der vorhergehenden Beschreibung mit einer Aussparung versehen ist, ermöglicht die Aussparung der Stützkomponente eine verbesserte Einsehbarkeit des distal/unter der Stützkomponente angeordneten Tibiaplateaus. Sofern die Rampe eine anteroposteriore Längsneigung aufweist, sind die beiden Rampenabschnitte mediolateral um die Abmessungen der Aussparung voneinander beabstandet. Folglich kann auch von einem medialen und einem lateralen Rampenabschnitt gesprochen werden. Sofern die Rampe eine mediolaterale Längsneigung aufweist, sind die beiden Rampenabschnitte um die Abmessungen der Aussparung anteroposterior voneinander beabstandet. In diesem Fall kann auch von einem anterioren Rampenabschnitt und einem posterioren Rampenabschnitt gesprochen werden. Beide Rampenabschnitte sind jeweils längserstreckt. Die Aussparung ist vorzugsweise in Längsrichtung der Rampe einends offen, wobei die Öffnung bevorzugt an einem niedrigen Ende der Rampe angeordnet ist.

In weiterer Ausgestaltung der Erfindung weist die wenigstens eine Stützkomponente, in proximaler und/oder distaler Blickrichtung, eine U-förmige Gestalt auf, wobei die beiden Rampenabschnitte jeweils einen längserstreckten Schenkel der U-Form bilden. Die U-Form der Stützkomponente ermöglicht insbesondere eine ergonomische Handhabung. In der Anwendung des tibialen Probeimplantats kann die U-förmige Stützkomponente im Bereich eines die beiden längserstreckten Schenkel verbindenden Querschenkels zwischen den Fingern einer Hand gegriffen werden. Dies ist ergonomisch vorteilhaft.

In weiterer Ausgestaltung der Erfindung sind die Artikulationskomponente und die wenigstens eine Stützkomponente jeweils aus einem biokompatiblen Kunststoffmaterial gefertigt. Biokompatible Kunststoffmaterialien sind der zuständigen Fachperson als solche bekannt. Die Verwendung solcher Kunststoffmaterialien für die Artikulationskomponente und die Stützkomponente bietet besondere Vorteile. Vorzugsweise sind die Artikulationskomponente und die wenigstens eine Stützkomponente jeweils einstückig aus dem besagten Kunststoffmaterial gefertigt. Die Fertigung kann beispielsweise mittels Spritzgießens erfolgen. Bei einer weiteren Ausgestaltung erfolgt stattdessen eine additive Fertigung.

In weiterer Ausgestaltung der Erfindung sind die Artikulationskomponente und die wenigstens eine Stützkomponente additiv mittels eines gemeinsamen 3D-Druckvorgangs gefertigt, wodurch die Artikulationskomponente und die wenigstens eine Stützkomponente über die Führungseinrichtung unlösbar miteinander verbunden und zueinander relativbeweglich sind. Bei dieser Ausgestaltung der Erfindung sind die Artikulationskomponente und die wenigstens eine Stützkomponente unverlierbar aneinander festgelegt. Durch den gemeinsamen 3D-Druckvorgang ist kein gesonderter Montageschritt zur Verbindung der Artikulationskomponente mit der wenigstens einen Stützkomponente erforderlich. Bevorzugt erfolgt der 3D-Druck unter Verwendung eines biokompatiblen Kunststoffmaterials.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Perspektivdarstellung eine Ausführungsform eines tibialen Probeimplantats mit einer Artikulationskomponente und einer Stützkomponente, wobei die Artikulationskomponente und die Stützkomponente relativ zueinander eine erste Konfiguration einnehmen,
- Fig. 2: das tibiale Probeimplantat nach Fig. 1 in einer schematischen Seitenansicht mit lateraler Blickrichtung,
- Fig. 3: das tibiale Probeimplantat nach den Fig. 1 und 2 in einer schematischen Draufsicht mit distaler Blickrichtung,
- Fig. 4: eine weitere schematische Perspektivdarstellung des tibialen Probeimplantats nach den Fig. 1 bis 3, wobei die Artikulationskomponente und die Stützkomponente relativ zueinander eine zweite Konfiguration einnehmen,
- Fig. 5, 6: das tibiale Probeimplantat in der Konfiguration gemäß Fig. 4 in einer schematischen Seitenansicht (Fig. 5) und einer schematischen Draufsicht (Fig. 6),
- Fig. 7: die Stützkomponente des tibialen Probeimplantats nach den Fig. 1 bis 6 in einer schematischen Perspektivansicht,
- Fig. 8: die Stützkomponente nach Fig. 7 in einer schematischen Seitenansicht,
- Fig. 9: in schematischer Perspektivdarstellung die Artikulationskomponente des tibialen Probeimplantats nach den Fig. 1 bis 6,
- Fig. 10: die Artikulationskomponente nach Fig. 9 in einer schematischen Vorderansicht mit posterior gerichteter Blickrichtung,
- Fig. 11: die Artikulationskomponente nach den Fig. 9 und 10 in einer schematischen Schnittdarstellung entlang eines Schnitts A-A gemäß Fig. 10, und
- Fig. 12, 13: schematische Perspektivdarstellungen einer weiteren Ausführungsform eines erfindungsgemäßen tibialen Probeimplantats in einer ersten Konfiguration (Fig. 12) und einer zweiten Konfiguration (Fig. 13).

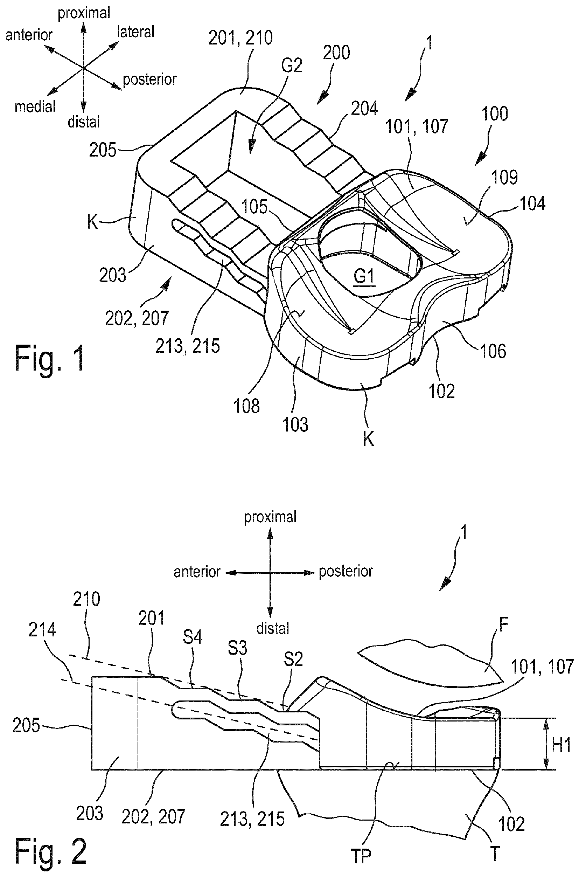

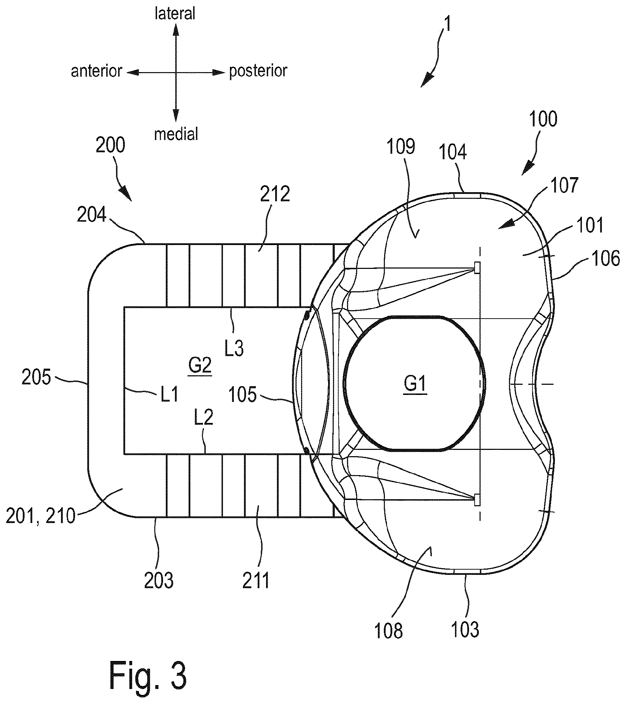

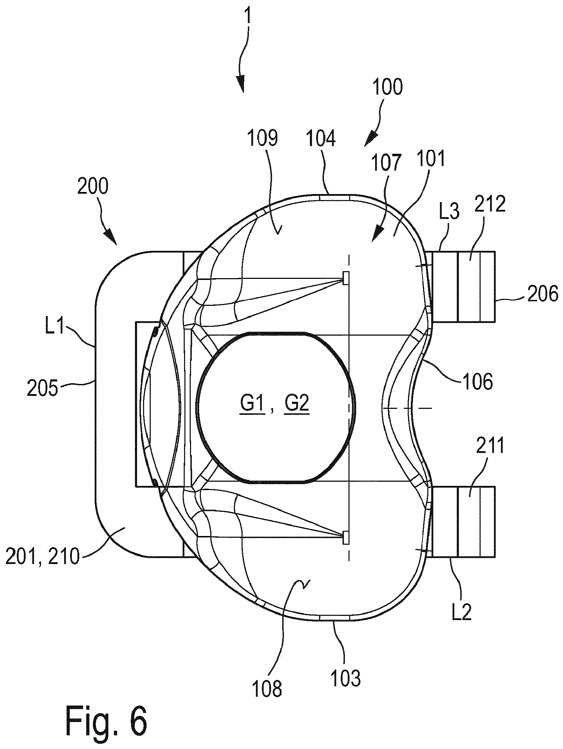

Gemäß den Fig. 1 bis 6 ist ein tibiales Probeimplantat 1 zur Verwendung bei einer Kniegelenkersatzoperation vorgesehen. Das tibiale Probeimplantat 1 dient der Ermittlung einer proximodistalen Höhe eines Tibiaimplantats und wird in der medizinischen Fachsprache oftmals auch als Probegleitfläche bezeichnet.

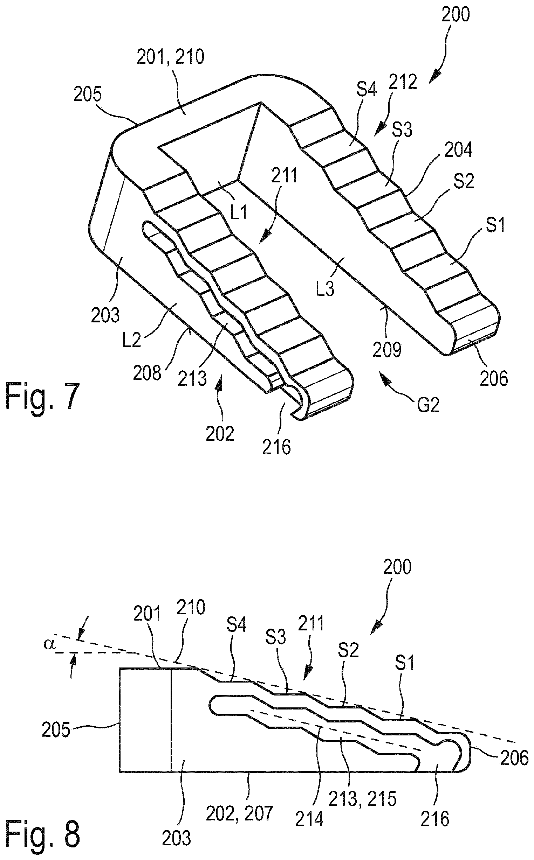

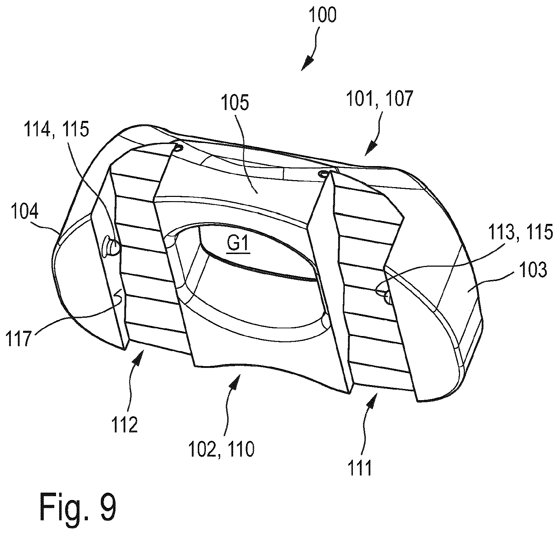

Das tibiale Probeimplantat 1 weist eine Artikulationskomponente 100 und eine Stützkomponente 200 auf. Die Artikulationskomponente 100 ist gesondert in den Fig. 9 bis 11 gezeigt. Die Stützkomponente ist gesondert in den Fig. 7 und 8 gezeigt.

Die Artikulationskomponente 100 weist eine proximale Oberseite 101, eine distale Unterseite 102, eine mediale Außenseite 103, eine laterale Außenseite 104, eine anteriore Vorderseite 105 und eine posteriore Rückseite 106 auf. Die proximale Oberseite 101 und die distale Unterseite 102 liegen entlang einer proximodistalen Achse einander gegenüber. Die mediale Außenseite 103 und die laterale Außenseite 104 liegen entlang einer mediolateralen Achse einander gegenüber. Die anteriore Vorderseite 105 und die posteriore Rückseite 106 liegen entlang einer anteroposterioren Achse einander gegenüber. Die besagten Achsen sind zueinander orthogonal.

Die Oberseite 101 der Artikulationskomponente 100 weist eine Artikulationsfläche 107 auf, die zur Artikulation mit einer femoralen Komponente F eingerichtet ist. Die femorale Komponente F ist in Fig. 2 schematisch und abgeschnitten gezeigt und kann durch einen natürlichen distalen Femur oder ein Femurimplantat gebildet sein.

Bei der gezeigten Ausführungsform ist die Artikulationsfläche 107 durch zwei Artikulationsflächenabschnitte 108, 109 gebildet, die in Bezug auf eine gedachte sagittale Mittellängsebene der Artikulationskomponente 100 einander gegenüberliegend angeordnet sind und auch als medialer Artikulationsflächenabschnitt 108 und lateraler Artikulationsflächenabschnitt 109 bezeichnet werden können.

Zwischen den Artikulationsflächenabschnitten 108, 109 weist die Artikulationskomponente 100 vorliegend eine proximodistal durchgängige Aussparung G1 auf. Die Aussparung G1 untergliedert die Artikulationsfläche 107 in die besagten Flächenabschnitte 108, 109 und ist als optional anzusehen. Die Aussparung G1 ist folglich nicht bei sämtlichen Ausführungsformen vorhanden.

Die Unterseite 102 der Artikulationskomponente 100 weist eine Stützfläche 110 auf (siehe insbesondere Fig. 9). Bei der gezeigten Ausführungsform weist die Stützfläche 110 zwei mediolateral voneinander beabstandete Stützflächenabschnitte 111, 112 auf, die auch als medialer Stützflächenabschnitt 111 und lateraler Stützflächenabschnitt 112 bezeichnet werden können. Eine solche Untergliederung/Teilung der Stützfläche 110 ist nicht bei sämtlichen Ausführungsformen vorgesehen und optional. Die besagte Aussparung G1 ist in Bezug auf die mediolaterale Achse zwischen den beiden Stützflächenabschnitten 111, 112 angeordnet.

Die Stützkomponente 200 weist eine proximale Oberseite 201, eine distale Unterseite 202, eine mediale Außenseite 203, eine laterale Außenseite 204, eine anteriore Vorderseite 205 und eine posteriore Rückseite 206 auf. Die proximale Oberseite 201 und die distale Unterseite 202 liegen entlang der proximodistalen Achse einander gegenüber. Die mediale Außenseite 203 und die laterale Außenseite 204 liegen entlang der mediolateralen Achse einander gegenüber. Die anteriore Vorderseite 205 und die posteriore Rückseite 206 liegen entlang der anteroposterioren Achse einander gegenüber.

Weiter weist die Stützkomponente 200 eine an der distalen Unterseite 202 angeordnete Lagerfläche 207 auf. Die Lagerfläche 207 ist zur Lagerung auf einem Tibiaplateau TP eingerichtet. Das TP ist schematisch in Fig. 2 gezeigt und vorliegend durch eine Resektionsfläche einer proximalen Tibia T gebildet. Alternativ kann das Tibiaplateau durch eine an der Resektionsfläche befestigte femorale Implantatskomponente gebildet sein. Die Lagerfläche 207 ist vorliegend parallel zu einer anteroposterior und mediolateral ausgedehnten Transversalebene.

Bei der gezeigten Ausführungsform weist die Stützkomponente 200 eine entlang der proximodistalen Achse durchgängige Aussparung G2 auf. Die Aussparung G2 unterteilt die Lagerfläche 207 in zwei mediolateral voneinander beabstandete Lagerflächenabschnitte 208, 209. Die Lagerflächenabschnitte 208, 209 können auch als medialer Lagerflächenabschnitt 208 und lateraler Lagerflächenabschnitt 209 bezeichnet werden. Die Aussparung G2 und die damit einhergehende Unterteilung der Lagerfläche 207 sind optional und folglich nicht bei sämtlichen Ausführungsformen vorhanden.

Weiter weist die Stützkomponente 200 eine proximale Rampe 210 auf. Die Rampe 210 bildet die Oberseite 201 und umgekehrt.

Bei der gezeigten Ausführungsform ist die Rampe 210 entlang der anteroposterioren Achse längsgeneigt und steigt ausgehend von der posterioren Rückseite 206 in Richtung der anterioren Vorderseite der Stützkomponente 200 an. Die Rampe 210 und/oder die Oberseite 201 ist folglich relativ zu der distalen Lagerfläche 207 geneigt. Die Längsneigung der Rampe 210 verläuft vorliegend in einem Winkel α gegenüber der Lagerfläche 207 und damit auch gegenüber dem Tibiaplateau TP.

Die bereits erläuterte Stützfläche 110 der Artikulationskomponente 100 (siehe Fig. 9) ist parallel zu der Rampe 210 längserstreckt und folglich entsprechend der Rampe 210 geneigt. Wie bereits erläutert, ist die Stützfläche 110 bei der gezeigten Ausführungsform durch die Stützflächenabschnitte 111, 112 gebildet, die folglich ebenfalls parallel zu der Rampe 210 parallel längserstreckt sind.

Bei der gezeigten Ausführungsform ist die Rampe 210 aufgrund der bereits erwähnten Aussparung G2 in zwei mediolateral voneinander beabstandete Rampenabschnitte 211, 212 untergliedert. Mit anderen Worten weist die Rampe 210 die beiden Rampenabschnitte 211, 212 auf, die auch als medialer Rampenabschnitt 211 und lateraler Rampenabschnitt 212 bezeichnet werden können. Eine solche Gestaltung der Rampe mit mehreren Rampenabschnitten ist optional und folglich nicht bei allen Ausführungsformen vorhanden.

Weiter weist die Stützkomponente 200 eine Führungseinrichtung 213 mit einer Führungsbahn 214 auf. Die Führungsbahn 214 ist in den Fig. 2 und 8 strichliert eingezeichnet. Die Führungsbahn 214 ist parallel zu der Rampe 210 längserstreckt.

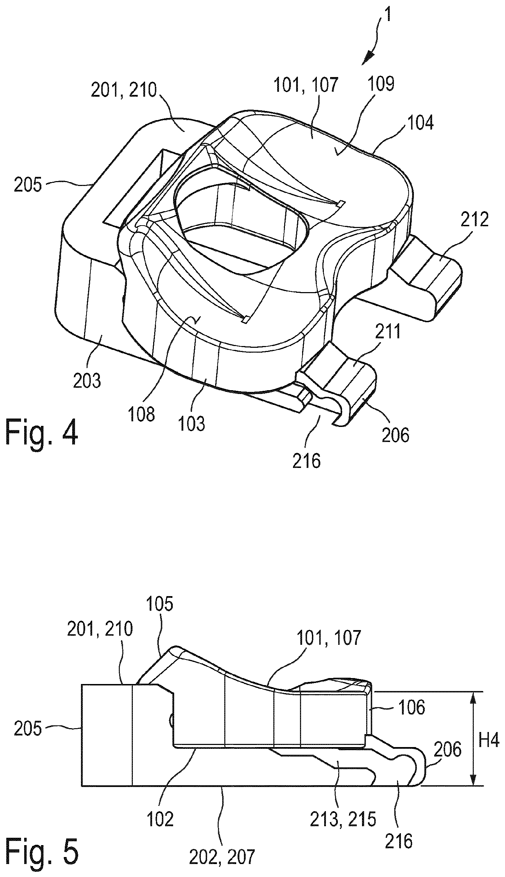

Die Artikulationskomponente 100 und die Stützkomponente 200 sind mittels der Führungseinrichtung 213 aneinander lineargeführt und relativ zueinander linearbeweglich. Die Linearbeweglichkeit verläuft entlang der Führungsbahn 214. Dabei sind die Artikulationskomponente 100 und die Stützkomponente 200 über die Stützfläche 110 und die Rampe 210 relativ zueinander beweglich aneinander abgestützt. Durch eine Relativbewegung zwischen der Artikulationskomponente 100 und der Stützkomponente 200 ist ein proximaler Abstand zwischen der Artikulationskomponente 100 und dem Tibiaplateau TP veränderlich. Die Relativbeweglichkeit erlaubt eine Veränderung des proximodistalen Abstands zwischen der Artikulationsfläche 107 der Artikulationskomponente 100 und der Lagerfläche 207 der Stützkomponente 200. Bei der in den Fig. 1 bis 3 gezeigten Konfiguration des tibialen Probeimplantats 1 ist ein proximodistaler Abstand H1 eingestellt. Die Artikulationskomponente 100, genauer: deren Stützfläche 110, ist hierfür vorliegend in einer in Bezug auf die Führungsbahn ersten Position relativ zu der Stützkomponente 200 auf der Rampe 210 abgestützt. Die in den Fig. 4 bis 6 gezeigte Konfiguration des tibialen Probeimplantats 1 wird bei einer hiervon unterschiedlichen relativen Positionierung der beiden Komponenten 100, 200 eingenommen. Dabei liegt ein unterschiedlicher proximodistaler Abstand H4 vor (siehe insbesondere Fig. 5).

Die Längsneigung der Rampe 210 und damit auch der Stützfläche 110 beträgt vorliegend in etwa 15°. Bei in den Figuren nicht gezeigten Ausführungsformen beträgt die Längsneigung zwischen 10° und 45°.

Intraoperativ erfolgt die Veränderung des proximodistalen Abstands bei der in den Fig. 1 bis 11 gezeigten Ausführungsform, indem der Operateur die Artikulationskomponente 100 ortsfest unterhalb der femoralen Komponente F hält und die Stützkomponente 200 entlang der anteroposterioren Achse relativ zu der Artikulationskomponente 100 und dem Tibiaplateau TP verlagert. Ausgehend von der in Fig. 2 gezeigten Konfiguration kann der proximodistale Abstand erhöht werden, indem die Stützkomponente 200 in posteriorer Richtung zwischen das Tibiaplateau TP und die Artikulationskomponente 100 vorgeschoben wird. Durch die rampenförmige Gestaltung der Oberseite 201 und die hierzu komplementäre Gestaltung der Stützfläche 110 ergibt sich in mediolateraler Blickrichtung eine keilförmige Gestalt der Stützkomponente 200. Die Stützkomponente 200 kann folglich auch als Keil bezeichnet werden. Zum Verringern des proximodistalen Abstands kann die Stützkomponente 200, beispielsweise ausgehend von der in Fig. 5 gezeigten Konfiguration, in anteriorer Richtung unter der ortsfest gehaltenen Artikulationskomponente 100 herausgezogen werden. Die Artikulationskomponente 100 und die Stützkomponente 200 sind bei einer solchen Verstellung des proximodistalen Abstands stets definiert aneinander geführt und/oder gehalten, insbesondere mittels der Führungseinrichtung 213.

Die Führungseinrichtung 213 weist bei der gezeigten Ausführungsform wenigstens eine Führungskulisse 215 auf. Die Führungskulisse 215 ist längserstreckt und definiert die Führungsbahn 214.

Bei der gezeigten Ausführungsform weist die Führungseinrichtung 213 zwei Führungskulissen 215 auf, wobei in den Figuren lediglich eine gezeigt ist. Die gezeigte Führungskulisse 215 ist in die mediale Außenseite 203 der Stützkomponente 200 eingesenkt. Die nicht gezeigte Führungskulisse ist in die laterale Außenseite 204 eingesenkt.

Bei der gezeigten Ausführungsform weist die Artikulationskomponente 100 wenigstens ein Führungselement 113, 114 zum Zusammenwirken mit der Führungseinrichtung 213 der Stützkomponente 200 auf. Vorliegend sind ein mediales Führungselement 113 und ein laterales Führungselement 114 vorhanden und jeweils als Führungsstift 115 gestaltet. Dabei greift das mediale Führungselement 113 in die mediale Führungskulisse 215 der Stützkomponente 200 ein. Das laterale Führungselement 114 greift in die (in den Figuren nicht gezeigte) laterale Führungskulisse ein. Die Führungsstifte 115 wirken entlang der Führungsbahn 214 gleitbeweglich mit den Führungskulissen zusammen.

Bei der in den Fig. 1 bis 11 gezeigten Ausführungsform sind die Führungskulissen 215 einends offen. Zur weiteren Erläuterung wird nachfolgend lediglich auf die mediale Führungskulisse 215 Bezug genommen. Hinsichtlich der nicht gezeigten lateralen Führungskulisse gilt, mutatis mutandis, Entsprechendes.

Die Führungskulisse 215 weist im Bereich der posterioren Rückseite 206 eine Einführöffnung 216 auf. Zwecks Montage des tibialen Probeimplantats 1 kann der mediale Führungsstift 113 durch die Einführöffnung 216 in die Führungskulisse 215 eingeführt werden.

Wie insbesondere in den Fig. 9 bis 11 gezeigt ist, ist die Stützfläche 110, genauer: die Stützflächenabschnitte 111, 112, proximal in die Unterseite 102 der Artikulationskomponente 100 eingesenkt. Die Stützfläche 110 ist folglich durch mediolateral gegenüberliegende Wandabschnitte 116, 117 begrenzt. Die besagten Wandabschnitte 116, 117 können auch als medialer Wandabschnitt 116 und lateraler Wandabschnitt 117 bezeichnet werden. Der mediale Wandabschnitt 116 liegt in lateraler Richtung formschlüssig an der medialen Außenseite 203 der Artikulationskomponente an. Der laterale Wandabschnitt 117 liegt in medialer Richtung formschlüssig an der lateralen Außenseite 204 der Stützkomponente 200 an.

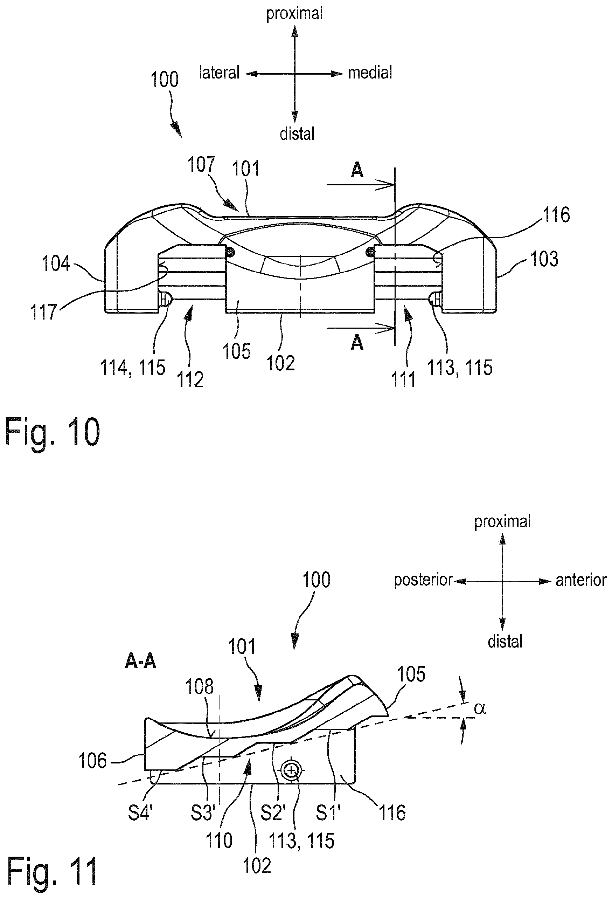

Bei der in den Fig. 1 bis 11 gezeigten Ausführungsform ist die Rampe 210 mit Stufen S1, S2, S3, S4 versehen (siehe Fig. 2). Entsprechendes gilt für die Stützfläche 110. Diese ist mit Stufen S1', S2', S3', S4' versehen (siehe Fig. 11). Aufgrund der spezifischen Gestaltung der vorliegenden Ausführungsform mit zwei Rampenabschnitten 211, 212 und zwei Stützflächenabschnitten 111, 112 weisen sämtliche jeweiligen Abschnitte eine abgestufte Gestaltung auf.

Durch die abgestufte Gestaltung der Rampe 210 und der Stützfläche 110 bewirkt eine Relativbewegung zwischen der Artikulationskomponente 100 und der Stützkomponente 200 eine stufenweise Veränderung des proximodistalen Abstands. Die Stufen S1 bis S4 der Stützkomponente 200 sind jeweils einem spezifischen proximodistalen Abstand zugeordnet. Die Stufe S1 definiert den proximodistalen Abstand H1 in der in den Fig. 1 bis 3 gezeigten Konfiguration. Die Stufe S4 definiert den (größeren) proximodistalen Abstand H4 der in den Fig. 4 bis 6 gezeigten Konfiguration. Die weiteren Stufen S2, S3 definieren zwischen den beiden proximodistalen Abständen H1, H4 liegende Abstände.

Die Stufen S1 bis S4 der Artikulationskomponente 200 steigen ausgehend von der posterioren Rückseite 206 in Richtung der anterioren Vorderseite 205 proximal an. Die Stufen S1' bis S4' der Artikulationskomponente 100 steigen ausgehend von der posterioren Rückseite 106 in Richtung der anterioren Vorderseite 105 distal an.

Es versteht sich, dass die Stufen S1 bis S4 und die Stufen S1' bis S4' zueinander komplementär gestaltet sind.

Um eine vereinfachte Relativbeweglichkeit zwischen den beiden Komponenten 100, 200 zu ermöglichen, sind die Stufen S1 bis S4 sowie S1' bis S4' jeweils angeschrägt. Infolge der Anschrägung der Stufen kann die Stützkomponente 200 auf die vorbeschriebene Weise relativ gleitbeweglich zu der Artikulationskomponente 100 anterior verlagert werden, ohne dass die Stufen S1 bis S4 der Stützkomponente 200 und die Stufen S1' bis S4' der Artikulationskomponente 200 aneinander verhaken.

Bei der gezeigten Ausführungsform beträgt die proximodistale Höhe zwischen den Stufen jeweils 2 mm. Bei in den Figuren nicht gezeigten Ausführungsformen beträgt die Höhe zwischen 1 mm und 3 mm. Vorliegend sind zudem jeweils vier Stufen vorhanden.

Bei einem Vorschub der Stützkomponente 200 unter die Artikulationskomponente 100 wird Letztere aufgrund der stufenweisen Gestaltung der Rampe 210 und der Stützfläche 110 schrittweise/stufenweise um jeweils 2 mm proximal angehoben. Bei einem anterioren Herausziehen verringert sich die Höhe der Artikulationskomponente 100 in Relation zu dem Tibiaplateau TP schrittweise um jeweils 2 mm.

Die Stufen S1 bis S4 und S1' bis S4' sind vorzugsweise mit Auswölbungen und komplementären Einwölbungen versehen, die lösbar Ineinandergreifen und einer ungewollten Relativbewegung zwischen der Artikulationskomponente und der Stützkomponente entgegenwirken. Die besagten Ein- und Auswölbungen sind in den Figuren nicht gesondert dargestellt.

Weiter sind die Artikulationskomponente 100 und die Stützkomponente 200 vorliegend jeweils aus einem biokompatiblen Kunststoffmaterial K gefertigt. Es sind unterschiedliche Fertigungsverfahren denkbar. Vorliegend erfolgt die Fertigung mittels 3D-Druck. Bei der in den Fig. 1 bis 11 gezeigten Ausführungsform werden die Artikulationskomponente 100 und die Stützkomponente 200 jeweils gesondert voneinander additiv hergestellt. Nach der additiven Fertigung der beiden Komponenten 100, 200 werden diese zu dem tibialen Probeimplantat 1 zusammengesetzt.

Bei der in den Fig. 1 bis 11 gezeigten Ausführungsform weist die Stützkomponente 200 eine U-förmige Gestalt auf. Die beiden Rampenabschnitte 211, 212 bilden jeweils einen längserstreckten Schenkel L2, L3 der besagten U-Form. Die U-Form weist zudem einen weiteren (kurzen) Schenkel L1 auf. Der weitere Schenkel L1 ist mediolateral längserstreckt endseitig der beiden Schenkel L2, L3 angeordnet und verbindet diese miteinander.

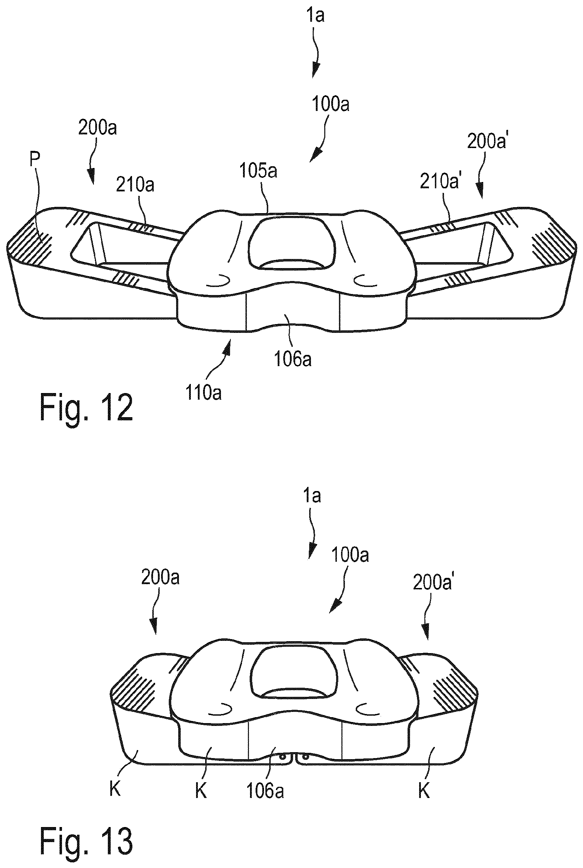

In den Fig. 12 und 13 ist eine weitere Ausführungsform eines erfindungsgemäßen tibialen Probeimplantats 1a gezeigt. Das tibiale Probeimplantat 1a ist hinsichtlich seines Aufbaus und seiner Funktion im Wesentlichen identisch mit dem tibialen Probeimplantat 1 nach den Fig. 1 bis 11. Zur Vermeidung von Wiederholungen wird daher nachfolgend lediglich auf wesentliche Unterschiede eingegangen. Funktionsgleiche Bauteile und/oder Abschnitte werden bei dem tibialen Probeimplantat 1a nach den Fig. 12 und 13 nicht gesondert erläutert. Stattdessen wird zur Vermeidung von Wiederholungen auf das bereits zu dem tibialen Probeimplantat 1 nach den Fig. 1 bis 11 Gesagte verwiesen und ausdrücklich Bezug genommen.

Wesentlicher Unterschied des tibialen Probeimplantats 1a ist das Vorhandensein zweier Stützkomponenten 200a, 200a'. Die beiden Stützkomponenten 200a, 200a' können auch als erste Stützkomponente 200a und zweite Stützkomponente 200a' bezeichnet werden und sind zueinander identisch gestaltet. Die beiden Stützkomponenten 200a, 200a' sind beidseits einer gedachten sagittalen Mittellängsebene der Artikulationskomponente 100a angeordnet und separat voneinander relativ zu der Artikulationskomponente 100a geführt linearbeweglich. Dabei verläuft die Neigung der jeweiligen Rampe 210a, 210a' jeweils in mediolateraler Richtung, so dass die Stützkomponenten 200a, 200a' nicht etwa anteroposterior, sondern stattdessen zueinander entgegengesetzt seitlich unter die Artikulationskomponente 100a geschoben werden können. Dabei zeigt Fig. 12 eine erste Konfiguration, in der ein minimaler proximodistaler Abstand eingestellt ist. Die Fig. 13 zeigt eine im Vergleich vergrößerte Höheneinstellung.

Weiter im Unterschied sind die jeweiligen Rampen 210a, 210a' - und damit auch die Stützfläche 110a der Artikulationskomponente 100a - bei dieser Ausführungsform nicht abgestuft. Stattdessen ist eine Oberflächenprofilierung P vorhanden. Die Oberflächenprofilierung P wirkt einem ungewollten Abrutschen der Artikulationskomponente 100a von den Stützkomponenten 200a, 200a' entgegen.

Auch die in den Fig. 12 und 13 gezeigte Ausführungsform ist additiv aus einem biokompatiblen Kunststoffmaterial K gefertigt. Die Fertigung des tibialen Probeimplantats 1a erfolgt hierbei mittels eines gemeinsamen 3D-Druckvorgangs. Infolge des gemeinsamen Druckvorgangs sind die Komponenten 100a, 200a, 200a' über die Führungseinrichtungen unlösbar miteinander verbunden und zugleich zueinander relativbeweglich.

## Patentansprüche

1. Tibiales Probeimplantat (1, 1a) zur Verwendung bei einer Kniegelenkersatzoperation, aufweisend
eine Artikulationskomponente (100, 100a) mit einer proximalen Artikulationsfläche (107), die zur Artikulation mit einer femoralen Komponente (F) eingerichtet ist, und mit einer distalen Unterseite (102),
wenigstens eine Stützkomponente (200, 200a, 200a') mit einer distalen Lagerfläche (207), die zur Lagerung auf einem Tibiaplateau (TP) eingerichtet ist, einer proximalen Oberseite (201), die eine relativ zu der distalen Lagerfläche (207) geneigte Rampe (210, 210a, 210a') bildet, und mit einer Führungseinrichtung (213), die eine parallel zu der Rampe (210, 210a, 210a') längserstreckte Führungsbahn (214) aufweist,
wobei die Unterseite (102) der Artikulationskomponente (100, 100a) eine parallel zu der Rampe (210, 210a, 210a') geneigte Stützfläche (110, 110a) aufweist,
und wobei die Artikulationskomponente (100, 100a) an der Führungseinrichtung (213) entlang der Führungsbahn (214) relativ zu der Stützkomponente (200, 200a, 200a') linearbeweglich mit ihrer Stützfläche (110, 110a) auf der Rampe (210, 210a, 210a') abgestützt ist,
wodurch eine Relativbewegung zwischen der Artikulationskomponente (100, 100a) und der Stützkomponente (200, 200a, 200a') eine Veränderung eines proximodistalen Abstands (H1, H4) zwischen der Artikulationsfläche (107) der Artikulationskomponente (100, 100a) und der Lagerfläche (207) der Stützkomponente (200, 200a, 200a') bewirkt,
**dadurch gekennzeichnet, dass** die Artikulationskomponente (100, 100a) an der Führungseinrichtung (213) gehalten ist.

2. Tibiales Probeimplantat (1, 1a) nach Anspruch 1, **dadurch gekennzeichnet, dass** die Rampe (210, 210a, 210a') und/oder die Stützfläche (110, 110a) plan ist und eine Oberflächenprofilierung (P) aufweist, die dazu eingerichtet ist, einem Abrutschen der Artikulationskomponente (100, 100a) von der Rampe (210, 210a, 210a') entgegenzuwirken.

3. Tibiales Probeimplantat (1) nach Anspruch 1 oder 2, **dadurch gekennzeichnet, dass** die Rampe (210) und die Stützfläche (110) jeweils mit einer Abfolge von Stufen (S1, S2, S3, S4; S1', S2', S3', S4') versehen sind, wodurch die Relativbewegung zwischen der Artikulationskomponente (100) und der Stützkomponente (200) eine stufenweise Veränderung des proximodistalen Abstands (H1, H4) bewirkt.

4. Tibiales Probeimplantat (1) nach Anspruch 3, **dadurch gekennzeichnet, dass** die Stufen (S1, S2, S3, S4; S1', S2', S3', S4') jeweils eine proximodistale Höhe zwischen 1 mm und 3 mm, bevorzugt 2 mm, aufweisen, wobei jeweils wenigstens vier Stufen vorhanden sind.

5. Tibiales Probeimplantat (1) nach Anspruch 3 oder 4, **dadurch gekennzeichnet, dass** die Stufen (S1, S2, S3, S4) der Rampe (210) jeweils eine konvexe Auswölbung aufweisen, und dass die Stufen (S1', S2', S3', S4') der Stützfläche (110) jeweils eine komplementäre konkave Einwölbung aufweisen, wobei die Auswölbungen und die Einwölbungen lösbar ineinandergreifen, wodurch einer ungewollten Relativbewegung zwischen der Artikulationskomponente (100) und der Stützkomponente (200) entgegengewirkt ist.

6. Tibiales Probeimplantat (1, 1a) nach einem der vorhergehenden Ansprüche, **dadurch gekennzeichnet, dass** die Rampe (210, 210a, 210a') und/oder die Stützfläche (110, 110a) eine Längsneigung (α) von 10° bis 45°, bevorzugt von 15° bis 30°, besonders bevorzugt von 20°, aufweist.

7. Tibiales Probeimplantat (1) nach einem der vorhergehenden Ansprüche, **dadurch gekennzeichnet, dass** die Rampe (210) und die Stützfläche (110) anteroposterior längsgeneigt sind.

8. Tibiales Probeimplantat (1a) nach einem der Ansprüche 1 bis 6, **dadurch gekennzeichnet, dass** die Rampe (210a, 210a') und die Stützfläche (110a) mediolateral längsgeneigt sind.

9. Tibiales Probeimplantat (1a) nach Anspruch 8, **dadurch gekennzeichnet, dass** zwei Stützkomponenten (200a, 200a') vorhanden und beidseits einer sagittalen Mittellängsebene der Artikulationskomponente (100a) angeordnet sind, wobei die beiden Stützkomponenten (200a, 200a') zur Veränderung des proximodistalen Abstands mediolateral entgegengesetzt relativ zu der Artikulationskomponente (100a) beweglich sind.

10. Tibiales Probeimplantat (1, 1a) nach einem der vorhergehenden Ansprüche, **dadurch gekennzeichnet, dass** die Führungseinrichtung (213) wenigstens eine Führungskulisse (215) aufweist, in welcher wenigstens ein Führungsstift (115) der Artikulationskomponente (100, 100a) entlang der Führungsbahn (214) gleitbeweglich eingreift, insbesondere wobei die Führungskulisse (215) einends der Führungsbahn (214) eine Einführöffnung (216) aufweist, die ausgehend von der Lagerfläche (207) im Wesentlichen senkrecht zu der Führungsbahn (214) in die Führungskulisse (215) mündet und durch welche der wenigstens eine Führungsstift (115) in die Führungskulisse (215) einführbar ist.

11. Tibiales Probeimplantat (1, 1a) nach einem der vorhergehenden Ansprüche, **dadurch gekennzeichnet, dass** die Stützfläche (110) proximal in die Unterseite (102) der Artikulationskomponente (100) eingesenkt und durch gegenüberliegende Wandabschnitte (116, 117) begrenzt ist, die jeweils senkrecht zu der Führungsbahn (214) und senkrecht zu einer proximodistalen Achse formschlüssig an der Stützkomponente (200) anliegen.

12. Tibiales Probeimplantat (1, 1a) nach einem der vorhergehenden Ansprüche, **dadurch gekennzeichnet, dass** die Artikulationskomponente (100) eine proximodistal von der Artikulationsfläche (107) bis zu der Stützfläche (110) erstreckte Aussparung (G1) aufweist, welche die Artikulationsfläche (107) und die Stützfläche (110) in jeweils einen medialen Flächenabschnitt (108, 111) und einen lateralen Flächenabschnitt (109, 112) teilt.

13. Tibiales Probeimplantat (1, 1a) nach einem der vorhergehenden Ansprüche, **dadurch gekennzeichnet, dass** die wenigstens eine Stützkomponente (200) eine proximodistal durch die Rampe (210) erstreckte Aussparung (G2) aufweist, welche die Rampe (210) in zwei Rampenabschnitte (211, 212) teilt, die seitlich, insbesondere mediolateral oder anteroposterior, voneinander beabstandet sind, insbesondere wobei die wenigstens eine Stützkomponente (200) - in proximaler und/oder distaler Blickrichtung - eine U-förmige Gestalt aufweist, wobei die beiden Rampenabschnitte (211, 212) jeweils einen längserstreckten Schenkel (L2, L3) der U-Form bilden.

14. Tibiales Probeimplantat (1a, 1a) nach einem der vorhergehenden Ansprüche, **dadurch gekennzeichnet, dass** die Artikulationskomponente (100, 100a) und die wenigstens eine Stützkomponente (200, 200a, 200a') jeweils aus einem biokompatiblen Kunststoffmaterial (K) gefertigt sind.

15. Tibiales Probeimplantat (1, 1a) nach einem der vorhergehenden Ansprüche, **dadurch gekennzeichnet, dass** die Artikulationskomponente (100, 100a) und die wenigstens eine Stützkomponente (200, 200a, 200a') additiv mittels eines gemeinsamen 3D-Druckvorgangs gefertigt sind, wodurch die Artikulationskomponente (100, 100a) und die wenigstens eine Stützkomponente (200, 200a, 200a') über die Führungseinrichtung (213) unlösbar miteinander verbunden und zueinander relativbeweglich sind.

## Claims

1. Tibial trial implant (1, 1a) for use in a knee joint replacement operation, having
an articulation component (100, 100a) with a proximal articulation surface (107) configured for articulation with a femoral component (F), and with a distal bottom side (102),
at least one support component (200, 200a, 200a') with a distal bearing surface (207) configured for bearing on a tibial plateau (TP), a proximal top side (201) forming a ramp (210, 210a, 210a') inclined relative to the distal bearing surface (207), and a guide device (213) having a guide track (210) extending longitudinally parallel to the ramp (210, 210a, 210a'),
wherein the bottom side (102) of the articulation component (100, 100a) has a support surface (110, 110a) inclined parallel to the ramp (210, 210a, 210a'),
and wherein the articulation component (100, 100a) is supported with its support surface (110, 110a) on the ramp (210, 210a, 210a') in a manner linearly movable along the guide track (214) relative to the support component (200, 200a, 200a'),
whereby a relative movement between the articulation component (100, 100a) and the support component (200, 200a, 200a') causes a change in a proximodistal distance (H1, H4) between the articulation surface (107) of the articulation component (100, 100a) and the bearing surface (207) of the support component (200, 200a, 200a'),
**characterized in that** the articulation component (100, 100a) is held on the guide device (213).

2. Tibial trial implant (1, 1a) according to Claim 1, **characterized in that** the ramp (210, 210a, 210a') and/or the support surface (110, 110a) is flat and has a surface profiling (P) configured to counteract slipping of the articulation component (100, 100a) from the ramp (210, 210a, 210a').

3. Tibial trial implant (1) according to Claim 1 or 2, **characterized in that** the ramp (210) and the support surface (110) are each provided with a sequence of steps (S1, S2, S3, S4; S1', S2', S3', S4'), whereby the relative movement between the articulation component (100) and the support component (200) causes a step-by-step change in the proximodistal distance (H1, H4).

4. Tibial trial implant (1) according to Claim 3, **characterized in that** the steps (S1, S2, S3, S4; S1', S2', S3', S4') each have a proximodistal height of between 1 mm and 3 mm, preferably 2 mm, with at least four steps each being present.

5. Tibial trial implant (1) according to Claim 3 or 4, **characterized in that** the steps (S1, S2, S3, S4) of the ramp (210) each have a convex protuberance, and **in that** the steps (S1', S2', S3', S4') of the support surface (110) each have a complementary concave indentation, wherein the protuberances and the indentations engage releasably in one another, thereby counteracting an unwanted relative movement between the articulation component (100) and the support component (200).

6. Tibial trial implant (1, 1a) according to one of the preceding claims, **characterized in that** the ramp (210, 210a, 210a') and/or the support surface (110, 110a) has a longitudinal inclination (α) of 10° to 45°, preferably of 15° to 30°, particularly preferably of 20°.

7. Tibial trial implant (1) according to one of the preceding claims, **characterized in that** the ramp (210) and the support surface (110) are longitudinally inclined in an anteroposterior direction.

8. Tibial trial implant (1a) according to one of Claims 1 to 6, **characterized in that** the ramp (210a, 210a') and the support surface (110a) are longitudinally inclined in a mediolateral direction.

9. Tibial trial implant (1a) according to Claim 8, **characterized in that** two support components (200a, 200a') are present and are arranged on both sides of a sagittal median longitudinal plane of the articulation component (100a), wherein the two support components (200a, 200a'), in order to change the proximodistal distance, are movable mediolaterally in opposite directions relative to the articulation component (100a).

10. Tibial trial implant (1, 1a) according to one of the preceding claims, **characterized in that** the guide device (213) has at least one guide slot (215), in which at least one guide pin (115) of the articulation component (100, 100a) engages slidably along the guide track (214), in particular wherein the guide slot (215) has, at one end of the guide track (214), an insertion opening (216) which, starting from the bearing surface (207), opens into the guide slot (215) substantially perpendicularly to the guide track (214), and through which the at least one guide pin (115) is insertable into the guide slot (215).

11. Tibial trial implant (1, 1a) according to one of the preceding claims, **characterized in that** that the support surface (110) is recessed proximally into the bottom side (102) of the articulation component (100) and is bounded by opposite wall sections (116, 117), which in each case bear with a form-fit on the support component (200) perpendicularly to the guide track (214) and perpendicularly to a proximodistal axis.

12. Tibial trial implant (1, 1a) according to one of the preceding claims, **characterized in that** that the articulation component (100) has a recess (G1) which extends proximodistally from the articulation surface (107) to the support surface (110) and which divides the articulation surface (107) and the support surface (110) into in each case a medial surface section (108, 111) and a lateral surface section (109, 112).

13. Tibial trial implant (1, 1a) according to one of the preceding claims, **characterized in that** the at least one support component (200) has a recess (G2) which extends proximodistally through the ramp (210) and which divides the ramp (210) into two ramp sections (211, 212), which are spaced apart from each other laterally, in particular mediolaterally or anteroposteriorly, in particular wherein the at least one support component (200), seen in a proximal and/or distal viewing direction, has a U-shaped configuration, wherein the two ramp sections (211, 212) each form an elongate leg (L2, L3) of the U-shape.

14. Tibial trial implant (1a, 1a) according to one of the preceding claims, **characterized in that** the articulation component (100, 100a) and the at least one support component (200, 200a, 200a') are each manufactured from a biocompatible plastic material (K).

15. Tibial trial implant (1, 1a) according to one of the preceding claims, **characterized in that** the articulation component (100, 100a) and the at least one support component (200, 200a, 200a') are additively manufactured by means of a single 3D printing process, whereby the articulation component (100, 100a) and the at least one support component (200, 200a, 200a') are connected nonreleasably to each other via the guide device (213) and are movable relative to each other.

## Revendications

1. Implant d'essai tibial (1, 1a) destiné à être utilisé lors d'une opération de remplacement de l'articulation du genou, présentant
une composante d'articulation (100, 100a) comportant une surface d'articulation proximale (107), configurée pour l'articulation avec une composante fémorale (F), et un côté inférieur distal (102),
au moins une composante de support (200, 200a, 200a') comportant une surface d'appui distale (207), configurée pour prendre appui sur un plateau tibial (TP), un côté supérieur proximal (201), qui forme une rampe (210, 210a, 210a') inclinée par rapport à la surface d'appui distale (207), et un dispositif de guidage (213) comportant une voie de guidage (214) s'étendant longitudinalement parallèlement à la rampe (210, 210a, 210a'),
le côté inférieur (102) de la composante d'articulation (100, 100a) comportant une surface de support (110, 110a) inclinée parallèlement à la rampe (210, 210a, 210a'),
et la composante d'articulation (100, 100a) étant supportée avec sa surface de support (110, 110a) sur la rampe (210, 210a, 210a') et étant mobile linéairement par rapport à la composante de support (200, 200a, 200a') le long de la voie de guidage (214) au niveau du dispositif de guidage (213),
de sorte qu'un mouvement relatif entre la composante d'articulation (100, 100a) et la composante de support (200, 200a, 200a') provoque une modification d'une distance proximodistale (H1, H4) entre la surface d'articulation (107) de la composante d'articulation (100, 100a) et la surface d'appui (207) de la composante de support (200, 200a, 200a'), **caractérisé en ce que** la composante d'articulation (100, 100a) est retenue au niveau du dispositif de guidage (213).

2. Implant d'essai tibial (1, 1a) selon la revendication 1, **caractérisé en ce que** la rampe (210, 210a, 210a') et/ou la surface de support (110, 110a) est plane et comporte un profilage de surface (P), configuré pour s'opposer à un glissement de la composante d'articulation (100, 100a) hors de la rampe (210, 210a, 210a').

3. Implant d'essai tibial (1) selon la revendication 1 ou 2, **caractérisé en ce que** la rampe (210) et la surface de support (110) sont chacune pourvues d'une succession de marches (S1, S2, S3, S4 ; S1', S2', S3', S4'), de sorte que le mouvement relatif entre la composante d'articulation (100) et la composante de support (200) provoque une modification par paliers de la distance proximodistale (H1, H4).

4. Implant d'essai tibial (1) selon la revendication 3, **caractérisé en ce que** les marches (S1, S2, S3, S4 ; S1', S2', S3', S4') présentent chacune une hauteur proximodistale comprise entre 1 mm et 3 mm, de préférence 2 mm, au moins quatre marches étant présentes dans chaque cas.

5. Implant d'essai tibial (1) selon la revendication 3 ou 4, **caractérisé en ce que** les marches (S1, S2, S3, S4) de la rampe (210) présentent chacune un bombement convexe, et **en ce que** les marches (S1', S2', S3', S4') de la surface de support (110) présentent chacune un évidement concave complémentaire, les bombements et les évidements s'emboîtant de manière amovible les uns dans les autres, de sorte qu'il est fait obstacle à un mouvement relatif non souhaité entre la composante d'articulation (100) et la composante de support (200).

6. Implant d'essai tibial (1, 1a) selon l'une quelconque des revendications précédentes, **caractérisé en ce que** la rampe (210, 210a, 210a') et/ou la surface de support (110, 110a) présente une inclinaison longitudinale (α) de 10° à 45°, de préférence de 15° à 30°, de manière particulièrement préférée de 20°.

7. Implant d'essai tibial (1) selon l'une quelconque des revendications précédentes, **caractérisé en ce que** la rampe (210) et la surface de support (110) présentent une inclinaison longitudinale antéropostérieure.

8. Implant d'essai tibial (1a) selon l'une quelconque des revendications 1 à 6, **caractérisé en ce que** la rampe (210a, 210a') et la surface de support (110a) présentent une inclinaison longitudinale médiolatérale.

9. Implant d'essai tibial (1a) selon la revendication 8, **caractérisé en ce que** deux composantes de support (200a, 200a') sont présentes et disposées de part et d'autre d'un plan sagittal médian longitudinal de la composante d'articulation (100a), les deux composantes de support (200a, 200a') étant mobiles, pour modifier la distance proximodistale, relativement à la composante d'articulation (100a) dans des directions médiolatérales opposées.

10. Implant d'essai tibial (1, 1a) selon l'une quelconque des revendications précédentes, **caractérisé en ce que** le dispositif de guidage (213) comporte au moins une coulisse de guidage (215), dans laquelle au moins une goupille de guidage (115) de la composante d'articulation (100, 100a) s'engage de manière coulissante le long de la voie de guidage (214), notamment la coulisse de guidage (215) comportant, à une extrémité de la voie de guidage (214), une ouverture d'introduction (216) débouchant dans la coulisse de guidage (215) à partir de la surface d'appui (207) essentiellement perpendiculairement à la voie de guidage (214), et par laquelle l'au moins une goupille de guidage (115) peut être introduite dans la coulisse de guidage (215).

11. Implant d'essai tibial (1, 1a) selon l'une quelconque des revendications précédentes, **caractérisé en ce que** la surface de support (110) est enfoncée proximalement dans le côté inférieur (102) de la composante d'articulation (100) et est délimitée par des sections de paroi opposées (116, 117), qui viennent chacune en appui par complémentarité de forme contre la composante de support (200), perpendiculairement à la voie de guidage (214) et perpendiculairement à un axe proximodistal.

12. Implant d'essai tibial (1, 1a) selon l'une quelconque des revendications précédentes, **caractérisé en ce que** la composante d'articulation (100) comporte un évidement (G1) s'étendant dans la direction proximodistale depuis la surface d'articulation (107) jusqu'à la surface de support (110), lequel divise la surface d'articulation (107) et la surface de support (110) en une section de surface médiale (108, 111) et une section de surface latérale (109, 112), respectivement.

13. Implant d'essai tibial (1, 1a) selon l'une quelconque des revendications précédentes, **caractérisé en ce que** l'au moins une composante de support (200) comporte un évidement (G2) s'étendant dans la direction proximodistale à travers la rampe (210), lequel divise la rampe (210) en deux sections de rampe (211, 212) espacées latéralement l'une de l'autre, notamment dans la direction médiolatérale ou antéropostérieure, notamment l'au moins une composante de support (200) présentant - dans une vue proximale et/ou distale - une forme en U, les deux sections de rampe (211, 212) formant chacune une branche longitudinale (L2, L3) de la forme en U.

14. Implant d'essai tibial (1a, 1a) selon l'une quelconque des revendications précédentes, **caractérisé en ce que** la composante d'articulation (100, 100a) et l'au moins une composante de support (200, 200a, 200a') sont chacune fabriquées à partir d'un matériau plastique biocompatible (K).

15. Implant d'essai tibial (1, 1a) selon l'une quelconque des revendications précédentes, **caractérisé en ce que** la composante d'articulation (100, 100a) et l'au moins une composante de support (200, 200a, 200a') sont fabriquées de manière additive au moyen d'un procédé commun d'impression 3D, de sorte que la composante d'articulation (100, 100a) et l'au moins une composante de support (200, 200a, 200a') sont reliées l'une à l'autre de manière indissociable via le dispositif de guidage (213) et sont mobiles l'une par rapport à l'autre.
